# EUROPEAN PATENT SPECIFICATION

(11) **EP 2 926 797 B1**
(45) Date of publication and mention of the grant of the patent: **06.09.2017**
(21) Application number: 14162818.0
(22) Date of filing: 31.03.2014
(51) Int. Cl.: A61K 8/24, A61Q 11/00, A61K 8/19

(54) **Surface-reacted calcium carbonate for remineralisation and whitening of teeth**
Oberflächenreaktives Calciumkarbonat zur Remineralisierung und Aufhellung von Zähnen
Carbonate de calcium traité par réaction en surface pour la reminéralisation et le blanchiment des dents

(43) Date of publication of application: 07.10.2015
(73) Proprietor: Omya International AG, 4665 Oftringen (CH)
(72) Inventor: BUDDE, Tanja, 4805 Brittnau (CH); Gerard, Daniel E., 4058 Basel (CH); Gane, Patrick A. C., 4852 Rothrist (CH)
(74) Representative: Müller-Dyck, Martina

(56) References cited:
- EP-A1- 2 264 108
- WO-A1-2010/037753
- US-A1- 2014 079 750

## Description

The present invention relates to new agents for remineralisation and whitening of teeth and oral care compositions including such agents and their use.

Tooth enamel is the hardest substance in the human body and contains about 96% minerals, wherein the remaining is composed of water and organic material. The primary mineral of enamel is hydroxylapatite, which is a crystalline calcium phosphate. Enamel is formed on the tooth while the tooth is developing within the gum, before it erupts into the mouth.

Its high mineral content makes the enamel, however, very vulnerable to a demineralisation process, which is especially triggered by the consumption of acidic drinks and sweets. Remineralisation of teeth can repair damage to the tooth to a certain degree but damage beyond that cannot be repaired by the body, and ultimately the continuing demineralisation process results in tooth erosion and dental caries. The maintenance and repair of human tooth enamel is therefore one of the primary concerns of dentistry.

A remineralisation study using a toothpaste containing hydroxylapatite and sodium monofluorophosphate is disclosed in Hornby et al., International Dental Journal 2009, 59, 325-331. US 2007/0183984 A1 is directed to an oral composition comprising a calcium phosphate salt and a combination of acids having different solubilities in the oral cavity, for tooth mineralisation or remineralisation.

The typical colour of enamel varies from light yellow to grayish or bluish white. Since enamel is semi-translucent, the colour of dentine and any material underneath the enamel strongly affects the appearance of a tooth. The enamel on primary teeth has a more opaque crystalline form and thus appears whiter than on permanent teeth. On radiographs, the differences in the mineralization of different portions of the tooth and surrounding periodontium can be noted; enamel appears lighter than dentin or pulp since it is denser than both and more radiopaque (cf. "Tooth enamel", Wikipedia, The Free Encyclopedia, 6 March 2014).

As a person ages the adult teeth often become darker due to changes in the mineral structure of the tooth. Furthermore, the teeth can become stained by bacterial pigments, food-goods and vegetables rich with carotenoids or xanthonoids. Certain antibacterial medications like tetracycline can cause teeth stains or a reduction in the brilliance of the enamel, and ingesting coloured liquids like coffee, tea, and red wine or smoking can discolour teeth ("Tooth bleaching", Wikipedia, The Free Encyclopedia, 5 February 2014).

Methods for whitening teeth often involve a bleaching process using aggressive oxidation agents such as peroxides, and may require the entire solid composition to remain in contact with the teeth for an extended period of time. As an alternative, dentifrice compositions providing both remineralisation and whitening of teeth employing calcium salts have been suggested.

WO 2012/143220 A1 describes a composition that is suitable for remineralisation and whitening of teeth, which comprises a calcium source and regeneration-source calcium salt. A dentifrice composition comprising a water insoluble and/or slightly water-soluble calcium source and an organic acid, or its physiologically acceptable salt, is described in WO 2013/034421 A2. WO 2012/031786 A2 relates to oral care compositions with composite particle actives having a core and a coating, whereby the coating interacts with phosphate ions to produce calcium and phosphate reaction products that are suitable to adhere to tooth enamel and/or dentine to improve the characteristics of teeth.

In view of the foregoing, there is a continuous need for agents that are useful in the remineralisation of teeth and/or whitening of teeth.

Accordingly, it is an object of the present invention to provide an agent that is suitable to remineralise and whiten teeth and is compatible with conventional oral care compositions. It would also be desirable to provide a remineralisation and/or whitening agent, which is gentle for use and easy to apply. It would also be desirable to provide a remineralisation and/or whitening agent, which does not necessarily require in-office treatments, but can be used at home, for example, on a daily basis.

It is also an object of the present invention to provide a remineralisation and/or whitening agent which is more resistant to acid challenge. It would also be desirable to provide a remineralisation and/or whitening agent which does not necessarily needs to a have a particle size in the nanosize range. It would also be desirable to provide a remineralisation and/or whitening agent which provides the additional benefit of being a carrier material for active agents.

The foregoing and other objects are solved by the subject-matter as defined herein in the independent claims.

According to one aspect of the present invention, a surface-reacted calcium carbonate for use in remineralisation and/or whitening of teeth is provided, wherein the surface-reacted calcium carbonate is a reaction product of natural or synthetic calcium carbonate with carbon dioxide and at least one acid.

According to another aspect of the present invention, an oral care composition for use in remineralisation and/or whitening of teeth is provided, comprising a surface-reacted calcium carbonate, wherein the surface-reacted calcium carbonate is a reaction product of natural or synthetic calcium carbonate with carbon dioxide and at least one acid.

According to still another aspect of the present invention, a surface-reacted calcium carbonate for use in remineralisation of teeth is provided, wherein the surface-reacted calcium carbonate is a reaction product of natural or synthetic calcium carbonate with carbon dioxide and at least one acid.

According to still another aspect of the present invention, a surface-reacted calcium carbonate for use in whitening of teeth enamel is provided, wherein the surface-reacted calcium carbonate is a reaction product of natural or synthetic calcium carbonate with carbon dioxide and at least one acid.

Advantageous embodiments of the present invention are defined in the corresponding sub-claims.

According to one embodiment the at least one acid is selected from the group consisting of hydrochloric acid, sulphuric acid, sulphurous acid, phosphoric acid, citric acid, oxalic acid, acetic acid, formic acid, and mixtures thereof, preferably the at least one acid is selected from the group consisting of hydrochloric acid, sulphuric acid, sulphurous acid, phosphoric acid, oxalic acid, and mixtures thereof, and more preferably the at least one acid is phosphoric acid.

According to one embodiment the surface-reacted calcium carbonate is in form of particles having a volume median grain diameter (*d₅₀*) of equal to or less than 15 µm, preferably from 1 to 10 µm, more preferably from 2 to 8 µm, and most preferably from 3 to 7 µm, and/or a volume determined top cut particle size (*d₉₈*) of equal to or less than 25 µm, preferably from 7 to 22 µm, more preferably from 10 to 20, and most preferably from 15 to 18 µm. According to another embodiment the surface-reacted calcium carbonate is in form of particles having a specific surface area of from 5 m²/g to 200 m²/g, more preferably 20 m²/g to 80 m²/g, and even more preferably 30 m²/g to 60 m²/g, measured using nitrogen and the BET method according to ISO 9277.

According to one embodiment the surface-reacted calcium carbonate is used in combination with a fluoride compound, preferably selected from the group consisting of sodium fluoride, stannous fluoride, sodium monofluorophosphate, potassium fluoride, potassium stannous fluoride, sodium fluorostannate, stannous chlorofluoride, amine fluoride, and mixtures thereof, and more preferably the fluoride compound is sodium monofluorophosphate and/or sodium fluoride. According to another embodiment at least one active agent is associated with the surface-reacted calcium carbonate, preferably the active agent is at least one additional desensitizing agent, and more preferably the at least one additional desensitizing agent is selected from the group consisting of potassium nitrate, gluteraldehyde, silver nitrate, zinc chloride, strontium chloride hexahydrate, sodium fluoride, stannous fluoride, strontium chloride, strontium acetate, arginine, hydroxylapatite, calcium sodium phosphosilicate, potassium oxalate, calcium phosphate, calcium carbonate, bioactive glasses, and mixtures thereof.

According to one embodiment the surface-reacted calcium carbonate is obtained by a process comprising the steps of: (a) providing a suspension of natural or synthetic calcium carbonate, (b) adding at least one acid having a pKₐ value of 0 or less at 20°C or having a pKₐ value from 0 to 2.5 at 20°C to the suspension of step a), and (c) treating the suspension of step (a) with carbon dioxide before, during or after step (b). According to another embodiment the surface-reacted calcium carbonate is obtained by a process comprising the steps of: (A) providing a natural or synthetic calcium carbonate, (B) providing at least one water-soluble acid, (C) providing gaseous CO₂, (D) contacting said natural or synthetic calcium carbonate of step (A) with the at least one acid of step (B) and with the CO₂ of step (C), characterised in that: (i) the at least one acid of step B) has a pKₐ of greater than 2.5 and less than or equal to 7 at 20°C, associated with the ionisation of its first available hydrogen, and a corresponding anion is formed on loss of this first available hydrogen capable of forming a water-soluble calcium salt, and (ii) following contacting the at least one acid with natural or synthetic calcium carbonate, at least one water-soluble salt, which in the case of a hydrogen-containing salt has a pKₐ of greater than 7 at 20°C, associated with the ionisation of the first available hydrogen, and the salt anion of which is capable of forming water-insoluble calcium salts, is additionally provided.

According to one embodiment the oral care composition comprises from 1 to 40 wt.-%, preferably from 1.5 to 35 wt.-%, more preferably from 2 to 30 wt.-% of the surface-reacted calcium carbonate, based on the total weight of the composition. According to another embodiment the oral care composition is a toothpaste, a toothpowder, or a mouthwash, and wherein preferably the surface-reacted calcium carbonate is a reaction product of natural or synthetic calcium carbonate with carbon dioxide and phosphoric acid.

According to one embodiment the oral composition further comprises a fluoride compound, preferably the fluoride compound is selected from the group consisting of sodium fluoride, stannous fluoride, sodium monofluorophosphate, potassium fluoride, potassium stannous fluoride, sodium fluorostannate, stannous chlorofluoride, amine fluoride, and mixtures thereof, and more preferably the fluoride compound is sodium monofluorophosphate and/or sodium fluoride. According to another embodiment the oral care composition further comprises an additional remineralisation and/or whitening agent, preferably selected from the group consisting of hydroxylapatite, e.g. nano-hydroxylapatite, calcium carbonate, e.g. amorphous calcium carbonate, and combinations thereof with casein phospholipids, hydrogen peroxide, carbamide peroxide, fluoride compounds, and mixtures thereof. According to still another embodiment the oral care composition has a pH between 7.5 and 10, preferably between 8 and 9.

It should be understood that for the purpose of the present invention, the following terms have the following meaning.

For the purpose of the present invention, an "acid" is defined as Brønsted-Lowry acid, that is to say, it is an H₃O⁺ ion provider. An "acid salt" is defined as an H₃O⁺ ion-provider, e.g., a hydrogen-containing salt, which is partially neutralised by an electropositive element. A "salt" is defined as an electrically neutral ionic compound formed from anions and cations. A "partially crystalline salt" is defined as a salt that, on XRD analysis, presents an essentially discrete diffraction pattern.

In accordance with the present invention, pKₐ, is the symbol representing the acid dissociation constant associated with a given ionisable hydrogen in a given acid, and is indicative of the natural degree of dissociation of this hydrogen from this acid at equilibrium in water at a given temperature. Such pKₐ values may be found in reference textbooks such as Harris, D. C. "Quantitative Chemical Analysis: 3rd Edition", 1991, W.H. Freeman & Co. (USA), ISBN 0-7167-2170-8.

"Ground calcium carbonate" (GCC) in the meaning of the present invention is a calcium carbonate obtained from natural sources, such as limestone, marble, dolomite, or chalk, and processed through a wet and/or dry treatment such as grinding, screening and/or fractionating, for example, by a cyclone or classifier.

"Precipitated calcium carbonate" (PCC) in the meaning of the present invention is a synthesised material, obtained by precipitation following reaction of carbon dioxide and lime in an aqueous, semi-dry or humid environment or by precipitation of a calcium and carbonate ion source in water. PCC may be in the vateritic, calcitic or aragonitic crystal form.

For the purpose of the present invention, a "surface-reacted calcium carbonate" is a material comprising calcium carbonate and an insoluble, at least partially crystalline, non-carbonate calcium salt, preferably, extending from the surface of at least part of the calcium carbonate. The calcium ions forming said at least partially crystalline non-carbonate calcium salt originate largely from the starting calcium carbonate material that also serves to form the surface-reacted calcium carbonate core. Such salts may include OH⁻ anions and/or crystal water.

In the meaning of the present invention "water-insoluble" materials are defined as materials which, when mixed with deionised water and filtered on a filter having a 0.2 µm pore size at 20°C to recover the liquid filtrate, provide less than or equal to 0.1 g of recovered solid material following evaporation at 95 to 100°C of 100 g of said liquid filtrate. "Water-soluble" materials are defined as materials leading to the recovery of greater than 0.1 g of recovered solid material following evaporation at 95 to 100°C of 100 g of said liquid filtrate.

Throughout the present document, the "particle size" of a calcium carbonate and other materials is described by its distribution of particle sizes. The value *dₓ* represents the diameter relative to which x % by weight of the particles have diameters less than *dₓ*. This means that the *d₂₀* value is the particle size at which 20 wt.-% of all particles are smaller, and the *d₇₅* value is the particle size at which 75 wt.-% of all particles are smaller. The *d₅₀* value is thus the weight median particle size, i.e. 50 wt.-% of all grains are bigger or smaller than this particle size. For the purpose of the present invention the particle size is specified as weight median particle size *d₅₀* unless indicated otherwise. For determining the weight median particle size *d₅₀* value a Sedigraph can be used. For the purpose of the present invention, the "particle size" of surface-reacted calcium is described as volume determined particle size distributions. For determining the volume determined particle size distribution, e.g., the volume median grain diameter (*d₅₀*) or the volume determined top cut particle size (*d₉₈*) of surface-reacted calcium carbonate, a Malvern Mastersizer 2000 can be used. The weight determined particle size distribution may correspond to the volume determined particle size if the density of all the particles is equal.

A "specific surface area (SSA)" of a calcium carbonate in the meaning of the present invention is defined as the surface area of the calcium carbonate divided by its mass. As used herein, the specific surface area is measured by nitrogen gas adsorption using the BET isotherm (ISO 9277:2010) and is specified in m²/g.

An "oral care composition" in the meaning of the present invention refers to a composition suitable for the use in the mouth and for veterinary and/or human applications but especially for use in applications for the human mouth.

For the purpose of the present invention, the term "viscosity" or "Brookfield viscosity" refers to Brookfield viscosity. The Brookfield viscosity is for this purpose measured by a Brookfield (Type RVT) viscometer at 20°C ± 2°C at 100 rpm using an appropriate spindle and is specified in mPa·s.

A "suspension" or "slurry" in the meaning of the present invention comprises insoluble solids and water, and optionally further additives, and usually contains large amounts of solids and, thus, is more viscous and can be of higher density than the liquid from which it is formed.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group, which preferably consists only of these embodiments.

Where an indefinite or definite article is used when referring to a singular noun, e.g. "a", "an" or "the", this includes a plural of that noun unless something else is specifically stated.

Terms like "obtainable" or "definable" and "obtained" or "defined" are used interchangeably. This e.g. means that, unless the context clearly dictates otherwise, the term "obtained" does not mean to indicate that e.g. an embodiment must be obtained by e.g. the sequence of steps following the term "obtained" though such a limited understanding is always included by the terms "obtained" or "defined" as a preferred embodiment.

According to the present invention, a surface-reacted calcium carbonate is used in remineralisation and/or whitening of teeth. The surface-reacted calcium carbonate is a reaction product of natural or synthetic calcium carbonate with carbon dioxide and at least one acid.

In the following the details and preferred embodiments of the inventive surface-reacted calcium carbonate will be set out in more details. It is to be understood that these technical details and embodiments also apply to the inventive method for producing the surface-reacted calcium carbonate as well as to the inventive compositions comprising the surface-reacted calcium carbonate.

### The surface-reacted calcium carbonate

According to the present invention, the surface-reacted calcium carbonate is a reaction product of natural or synthetic calcium carbonate with carbon dioxide and at least one acid.

Natural (or ground) calcium carbonate (GCC) is understood to be a naturally occurring form of calcium carbonate, mined from sedimentary rocks such as limestone or chalk, or from metamorphic marble rocks. Calcium carbonate is known to exist mainly as three types of crystal polymorphs: calcite, aragonite and vaterite. Calcite, the most common crystal polymorph, is considered to be the most stable crystal form of calcium carbonate. Less common is aragonite, which has a discrete or clustered needle orthorhombic crystal structure. Vaterite is the rarest calcium carbonate polymorph and is generally unstable. Natural calcium carbonate is almost exclusively of the calcitic polymorph, which is said to be trigonal-rhombohedral and represents the most stable of the calcium carbonate polymorphs. The term "source" of the calcium carbonate in the meaning of the present invention refers to the naturally occurring mineral material from which the calcium carbonate is obtained. The source of the calcium carbonate may comprise further naturally occurring components such as magnesium carbonate, alumino silicate etc.

According to one embodiment of the present invention, the natural calcium carbonate is selected from the group consisting of marble, chalk, dolomite, limestone and mixtures thereof.

According to one embodiment of the present invention the GCC is obtained by dry grinding. According to another embodiment of the present invention the GCC is obtained by wet grinding and optionally subsequent drying.

In general, the grinding step can be carried out with any conventional grinding device, for example, under conditions such that comminution predominantly results from impacts with a secondary body, i.e. in one or more of: a ball mill, a rod mill, a vibrating mill, a roll crusher, a centrifugal impact mill, a vertical bead mill, an attrition mill, a pin mill, a hammer mill, a pulveriser, a shredder, a de-clumper, a knife cutter, or other such equipment known to the skilled man. In case the calcium carbonate containing mineral material comprises a wet ground calcium carbonate containing mineral material, the grinding step may be performed under conditions such that autogenous grinding takes place and/or by horizontal ball milling, and/or other such processes known to the skilled man. The wet processed ground calcium carbonate containing mineral material thus obtained may be washed and dewatered by well-known processes, e.g. by flocculation, filtration or forced evaporation prior to drying. The subsequent step of drying may be carried out in a single step such as spray drying, or in at least two steps. It is also common that such a mineral material undergoes a beneficiation step (such as a flotation, bleaching or magnetic separation step) to remove impurities.

"Precipitated calcium carbonate" (PCC) in the meaning of the present invention is a synthesized material, generally obtained by precipitation following reaction of carbon dioxide and lime in an aqueous environment or by precipitation of a calcium and carbonate ion source in water or by precipitation of calcium and carbonate ions, for example CaCl₂ and Na₂CO₃, out of solution. Further possible ways of producing PCC are the lime soda process, or the Solvay process in which PCC is a by-product of ammonia production. Precipitated calcium carbonate exists in three primary crystalline forms: calcite, aragonite and vaterite, and there are many different polymorphs (crystal habits) for each of these crystalline forms. Calcite has a trigonal structure with typical crystal habits such as scalenohedral (S-PCC), rhombohedral (R-PCC), hexagonal prismatic, pinacoidal, colloidal (C-PCC), cubic, and prismatic (P-PCC). Aragonite is an orthorhombic structure with typical crystal habits of twinned hexagonal prismatic crystals, as well as a diverse assortment of thin elongated prismatic, curved bladed, steep pyramidal, chisel shaped crystals, branching tree, and coral or worm-like form. Vaterite belongs to the hexagonal crystal system. The obtained PCC slurry can be mechanically dewatered and dried.

According to one embodiment of the present invention, the synthetic calcium carbonate is precipitated calcium carbonate, preferably comprising aragonitic, vateritic or calcitic mineralogical crystal forms or mixtures thereof.

According to one embodiment of the present invention, the natural or synthetic calcium carbonate is ground prior to the treatment with carbon dioxide and at least one acid. The grinding step can be carried out with any conventional grinding device such as a grinding mill known to the skilled person.

According to one embodiment of the present invention, the natural or synthetic calcium carbonate is in form of particles having a weight median particle size *d*₅₀ of equal to or less than 15 µm, preferably from 1 to 10 µm, more preferably from 2 to 8 µm, and most preferably from 3 to 7 µm. According to a further embodiment of the present invention, the natural or synthetic calcium carbonate is in form of particles having a top cut particle size *d*₉₈ of equal to or less than 25 µm, preferably from 7 to 22 µm, more preferably from 10 to 20 µm, and most preferably from 15 to 18 µm.

Preferably the surface-reacted calcium carbonate to be used in the present invention is prepared as an aqueous suspension having a pH, measured at 20°C, of greater than 6.0, preferably greater than 6.5, more preferably greater than 7.0, even more preferably greater than 7.5.

In a preferred process for the preparation of the aqueous suspension of surface-reacted calcium carbonate, the natural or synthetic calcium carbonate, either finely divided, such as by grinding, or not, is suspended in water. Preferably, the slurry has a content of natural or synthetic calcium carbonate within the range of 1 wt.-% to 90 wt.-%, more preferably 3 wt.-% to 60 wt.-%, and even more preferably 5 wt.-% to 40 wt.-%, based on the weight of the slurry.

In a next step, at least one acid is added to the aqueous suspension containing the natural or synthetic calcium carbonate. The at least one acid can be any strong acid, medium-strong acid, or weak acid, or mixtures thereof, generating H₃O⁺ ions under the preparation conditions. According to the present invention, the at least one acid can also be an acidic salt, generating H₃O⁺ ions under the preparation conditions.

According to one embodiment, the at least one acid is a strong acid having a pKₐ of 0 or less at 20°C. According to another embodiment, the at least one acid is a medium-strong acid having a pKₐ value from 0 to 2.5 at 20°C. If the pKₐ at 20°C is 0 or less, the acid is preferably selected from sulphuric acid, hydrochloric acid, or mixtures thereof. If the pKₐ at 20°C is from 0 to 2.5, the acid is preferably selected from H₂SO₃, H₃PO₄, oxalic acid, or mixtures thereof. The at least one acid can also be an acidic salt, for example, HSO₄⁻ or H₂PO₄⁻, being at least partially neutralized by a corresponding cation such as Li⁺, Na⁺ or K⁺, or HPO₄²⁻, being at least partially neutralised by a corresponding cation such as Li⁺, Na⁺, K⁺, Mg²⁺ or Ca²⁺. The at least one acid can also be a mixture of one or more acids and one or more acidic salts.

According to still another embodiment, the at least one acid is a weak acid having a pKₐ value of greater than 2.5 and less than or equal to 7, when measured at 20°C, associated with the ionisation of the first available hydrogen, and having a corresponding anion formed on loss of this first available hydrogen, which is capable of forming water-soluble calcium salts. According to the preferred embodiment, the weak acid has a pKₐ value from 2.6 to 5 at 20°C, and more preferably the weak acid is selected from the group consisting of acetic acid, formic acid, propanoic acid, and mixtures thereof.

In case a weak acid is used, after addition of said acid to the aqueous suspension containing the natural or synthetic calcium carbonate, at least one water-soluble salt, which in the case of a hydrogen-containing salt has a pKₐ of greater than 7, when measured at 20°C, associated with the ionisation of the first available hydrogen, and the salt anion of which is capable of forming water-insoluble calcium salts, is additionally added. The cation of said water-soluble salt is preferably selected from the group consisting of potassium, sodium, lithium and mixtures thereof. In a more preferred embodiment, said cation is sodium. It is of note that depending on the charge of the anion, more than one of said cations may be present to provide an electrically neutral ionic compound. The anion of said water-soluble salt is preferably selected from the group consisting of phosphate, dihydrogen phosphate, monohydrogen phosphate, oxalate, silicate, mixtures thereof and hydrates thereof. In a more preferred embodiment, said anion is selected from the group consisting of phosphate, dihydrogen phosphate, monohydrogen phosphate, mixtures thereof and hydrates thereof. In a most preferred embodiment, said anion is selected from the group consisting of dihydrogen phosphate, monohydrogen phosphate, mixtures thereof and hydrates thereof. Water-soluble salt addition may be performed dropwise or in one step. In the case of drop wise addition, this addition preferably takes place within a time period of 10 minutes. It is more preferred to add said salt in one step.

According to one embodiment of the present invention, the at least one acid is selected from the group consisting of hydrochloric acid, sulphuric acid, sulphurous acid, phosphoric acid, citric acid, oxalic acid, acetic acid, formic acid, and mixtures thereof. Preferably the at least one acid is selected from the group consisting of hydrochloric acid, sulphuric acid, sulphurous acid, phosphoric acid, oxalic acid, H₂PO₄⁻, being at least partially neutralised by a corresponding cation such as Li⁺, Na⁺ or K⁺, HPO₄²⁻, being at least partially neutralised by a corresponding cation such as Li⁺, Na⁺, K⁺, Mg²⁺ or Ca²⁺, and mixtures thereof, more preferably the at least one acid is selected from the group consisting of hydrochloric acid, sulphuric acid, sulphurous acid, phosphoric acid, oxalic acid, or mixtures thereof, and most preferably, the at least one acid is phosphoric acid. Without being bound to any theory, the inventors believe that the use of phosphoric acid can be beneficial in remineralisation and/or whitening of teeth.

The at least one acid can be added to the suspension as a concentrated solution or a more diluted solution. Preferably, the molar ratio of the at least one acid to the natural or synthetic calcium carbonate is from 0.05 to 4, more preferably from 0.1 to 2.

As an alternative, it is also possible to add the at least one acid to the water before the natural or synthetic calcium carbonate is suspended.

According to the present invention, the surface-reacted calcium carbonate is obtained by treating the natural or synthetic calcium carbonate with carbon dioxide. The carbon dioxide can be formed in situ by the acid treatment and/or can be supplied from an external source. If a strong acid such as sulphuric acid or hydrochloric acid or medium-strong acid such as phosphoric acid is used for the acid treatment of the natural or synthetic calcium carbonate, the carbon dioxide is automatically formed. Alternatively or additionally, the carbon dioxide can be supplied from an external source.

According to one embodiment, the surface-reacted calcium carbonate is a reaction product of natural or synthetic calcium carbonate with carbon dioxide and at least one acid, wherein the carbon dioxide is formed in situ as a result of contacting the at least one acid with the natural or synthetic calcium carbonate and/or is supplied from an external source.

Acid treatment and treatment with carbon dioxide can be carried out simultaneously which is the case when a strong or medium-strong acid is used. It is also possible to carry out acid treatment first, e.g. with a medium strong acid having a pKₐ in the range of 0 to 2.5 at 20°C, wherein carbon dioxide is formed in situ, and thus, the carbon dioxide treatment will automatically be carried out simultaneously with the acid treatment, followed by the additional treatment with carbon dioxide supplied from an external source.

Preferably, the concentration of gaseous carbon dioxide in the suspension is, in terms of volume, such that the ratio (volume of suspension):(volume of gaseous CO₂) is from 1:0.05 to 1:20, even more preferably from 1:0.05 to 1:5.

In a preferred embodiment, the acid treatment step and/or the carbon dioxide treatment step are repeated at least once, more preferably several times. According to one embodiment, the at least one acid is added over a time period of at least 30 min, preferably at least 45 min, and more preferably at least 1 h.

Subsequent to the acid treatment and carbon dioxide treatment, the pH of the aqueous suspension, measured at 20°C, naturally reaches a value of greater than 6.0, preferably greater than 6.5, more preferably greater than 7.0, even more preferably greater than 7.5, thereby preparing the surface-reacted calcium carbonate as an aqueous suspension having a pH of greater than 6.0, preferably greater than 6.5, more preferably greater than 7.0, even more preferably greater than 7.5. If the aqueous suspension is allowed to reach equilibrium, the pH is greater than 7. A pH of greater than 6.0 can be adjusted without the addition of a base when stirring of the aqueous suspension is continued for a sufficient time period, preferably 1 hour to 10 hours, more preferably 1 to 5 hours.

Alternatively, prior to reaching equilibrium, which occurs at a pH greater than 7, the pH of the aqueous suspension may be increased to a value greater than 6 by adding a base subsequent to carbon dioxide treatment. Any conventional base such as sodium hydroxide or potassium hydroxide can be used.

Further details about the preparation of the surface-reacted natural calcium carbonate are disclosed in WO 00/39222 A1 and US 2004/0020410 A1, wherein the surface-reacted natural calcium carbonate is described as a filler for paper manufacture. The preparation of surface-reacted calcium carbonate with weak acids is disclosed in EP 2 264 108 A1. The preparation of surface-reacted calcium carbonate and its use in purification processes is disclosed in EP 1 974 806 A1, EP 1 982 759 A1, and EP 1 974 807 A1. The use of surface-reacted calcium carbonate as carrier for the controlled release of active agents is described in WO 2010/037753 A1.

Similarly, surface-reacted precipitated calcium carbonate is obtained. As can be taken in detail from EP 2 070 991 A1, surface-reacted precipitated calcium carbonate is obtained by contacting precipitated calcium carbonate with H₃O⁺ ions and with anions being solubilised in an aqueous medium and being capable of forming water-insoluble calcium salts, in an aqueous medium to form a slurry of surface-reacted precipitated calcium carbonate, wherein said surface-reacted precipitated calcium carbonate comprises an insoluble, at least partially crystalline calcium salt of said anion formed on the surface of at least part of the precipitated calcium carbonate.

Said solubilised calcium ions correspond to an excess of solubilised calcium ions relative to the solubilised calcium ions naturally generated on dissolution of precipitated calcium carbonate by H₃O⁺ ions, where said H₃O⁺ ions are provided solely in the form of a counterion to the anion, i.e. via the addition of the anion in the form of an acid or non-calcium acid salt, and in absence of any further calcium ion or calcium ion generating source.

Said excess solubilised calcium ions are preferably provided by the addition of a soluble neutral or acid calcium salt, or by the addition of an acid or a neutral or acid non-calcium salt which generates a soluble neutral or acid calcium salt in situ.

Said H₃O⁺ ions may be provided by the addition of an acid or an acid salt of said anion, or the addition of an acid or an acid salt which simultaneously serves to provide all or part of said excess solubilised calcium ions.

According to one embodiment of the present invention, the surface-reacted calcium carbonate is obtained by a process comprising the steps of:
a) providing a suspension of natural or synthetic calcium carbonate,
b) adding at least one acid having a pKₐ value of 0 or less at 20°C or having a pKₐ value from 0 to 2.5 at 20°C to the suspension of step a), and
c) treating the suspension of step a) with carbon dioxide before, during or after step b).

According to one embodiment, at least one acid having a pKₐ value of 0 or less at 20°C is added in step b) to the suspension of step a). According to another embodiment, at least one acid having a pKₐ value from 0 to 2.5 at 20°C is added in step b) to the suspension of step a).

The carbon dioxide used in step c) can be formed in situ by the acid treatment of step b) and/or can be supplied from an external source.

According to one embodiment of the present invention, the surface-reacted calcium carbonate is obtained by a process comprising the steps of:
A) providing a natural or synthetic calcium carbonate,
B) providing at least one water-soluble acid,
C) providing gaseous CO₂,
D) contacting said natural or synthetic calcium carbonate of step A) with the at least one acid of step B) and with the CO₂ of step C),
characterised in that:
i) the at least one acid of step B) has a pKₐ of greater than 2.5 and less than or equal to 7 at 20°C, associated with the ionisation of its first available hydrogen, and a corresponding anion is formed on loss of this first available hydrogen capable of forming a water-soluble calcium salt, and
ii) following contacting the at least one acid with natural or synthetic calcium carbonate, at least one water-soluble salt, which in the case of a hydrogen-containing salt has a pKₐ of greater than 7 at 20°C, associated with the ionisation of the first available hydrogen, and the salt anion of which is capable of forming water-insoluble calcium salts, is additionally provided.

The surface-reacted calcium carbonate can be kept in suspension, optionally further stabilised by a dispersant. Conventional dispersants known to the skilled person can be used. A preferred dispersant is polyacrylic acid and/or carboxymethylcellulose.

Alternatively, the aqueous suspension described above can be dried, thereby obtaining the solid (i.e. dry or containing as little water that it is not in a fluid form) surface-reacted natural or synthetic calcium carbonate in the form of granules or a powder.

According to one embodiment of the present invention, the surface-reacted calcium carbonate has a specific surface area of from 5 m²/g to 200 m²/g, more preferably 20 m²/g to 80 m²/g and even more preferably 30 m²/g to 60 m²/g, measured using nitrogen and the BET method according to ISO 9277.

According to one embodiment of the present invention, the surface-reacted calcium carbonate is in form of particles having a volume median grain diameter (*d*₅₀) of equal to or less than 15 µm, preferably from 1 to 10 µm, more preferably from 2 to 8 µm, and most preferably from 3 to 7 µm. According to another embodiment of the present invention, the surface-reacted calcium carbonate is in form of particles having a volume determined top cut particle size (*d*₉₈) of equal to or less than 25 µm, preferably from 7 to 22 µm, more preferably from 10 to 20 µm, and most preferably from 15 to 18 µm. According to a preferred embodiment of the present invention, the surface-reacted calcium carbonate is in form of particles having volume median grain diameter (*d*₅₀) of equal to or less than 15 µm, preferably from 1 to 10 µm, more preferably from 2 to 8 µm, and most preferably from 3 to 7 µm, and having a volume determined top cut particle size (*d*₉₈) of equal to or less than 25 µm, preferably from 7 to 22 µm, more preferably from 10 to 20 µm, and most preferably from 15 to 18 µm The volume median grain diameter (*d*₅₀) and volume determined top cut particle size (*d*₉₈) can be determined by laser diffraction measurements, for example, by using a Malvern Mastersizer 2000.

According to one embodiment of the present invention, the surface-reacted calcium carbonate comprises an insoluble, at least partially crystalline calcium salt of an anion of the at least one acid, which is formed on the surface of the natural or synthetic calcium carbonate. According to one embodiment, the insoluble, at least partially crystalline salt of an anion of the at least one acid covers the surface of the natural or synthetic calcium carbonate at least partially, preferably completely. Depending on the employed at least one acid, the anion may be sulphate, sulphite, phosphate, citrate, oxalate, acetate, formiate and/or chloride.

According to one preferred embodiment, the surface-reacted calcium carbonate is a reaction product of natural calcium carbonate and at least one acid, preferably phosphoric acid.

The surface-reacted calcium carbonate has a good loading capacity and can be used as a carrier in oral care. For example, the surface-reacted calcium carbonate is capable of associating and transporting an active agent. The association preferably is an adsorption onto the surface of the surface-reacted calcium carbonate particles, be it the outer or the inner surface of the particles or an absorption into the particles, which is possible due to their porosity.

In this respect, it is believed that because of the intra and interpore structure of the surface-reacted calcium carbonate, this material is a superior agent to deliver previously ad/absorbed materials over time relative to common materials having similar specific surface areas.

The surface-reacted calcium carbonate may have an intra particle porosity within the range from 5 vol.-% to 50 vol.-%, preferably from 20 vol.-% to 50 vol.-%, and more preferably from 30 vol.-% to 50 vol.-%, calculated from mercury porosimetry measurement. From the bimodal derivative pore size distribution curve the lowest point between the peaks indicates the diameter where the intra and inter-particle pore volumes can be separated. The pore volume at diameters greater than this diameter is the pore volume associated with the inter-particle pores. The total pore volume minus this inter particle pore volume gives the intra particle pore volume from which the intra particle porosity can be calculated, preferably as a fraction of the solid material volume, as described in Transport in Porous Media (2006) 63: 239-259.
Further details with respect to the porosity of the surface-reacted calcium carbonate and its use as agent for delivering materials can be found in WO 2010/037753 A1.

Thus, generally, any agent fitting into the intra- and/or inter particle pores of the surface-reacted calcium carbonate is suitable to be transported by the surface- reacted calcium carbonate according to the invention. For example, active agents such as those selected from the group comprising pharmaceutically active agents, biologically active agents, disinfecting agents, preservatives such as triclosan, flavouring agents, surfactants like defoamers, or additional desensitizing agents can be used. According to one embodiment, at least one active agent is associated with the surface-reacted calcium carbonate. According to a preferred embodiment the active agent is at least one additional desensitising agent, preferably selected from the group consisting of potassium nitrate, gluteraldehyde, silver nitrate, zinc chloride, strontium chloride hexahydrate, sodium fluoride, stannous fluoride, strontium chloride, strontium acetate, arginine, hydroxylapatite, calcium sodium phosphosilicate, potassium oxalate, calcium phosphate, calcium carbonate, bioactive glasses, and mixtures thereof. Hydroxylapatite, also called hydroxyapatite, is a naturally occurring mineral form of calcium apatite with the formula Ca₅(PO₄)₃(OH). According to an exemplary embodiment, the hydroxylapatite is a nanosized hydroxylapatite, also called nano-hydoxylapatite.

### The oral care composition

The oral care composition for the use according to the present invention comprises a surface-reacted calcium carbonate, wherein the surface-reacted calcium carbonate is a reaction product of natural or synthetic calcium carbonate with carbon dioxide and at least one acid.

According to one embodiment of the present invention, the composition comprises from 1 to 40 wt.-%, preferably from 1.5 to 35 wt.-%, more preferably from 2 to 30 wt.-% of the surface-reacted calcium carbonate, based on the total weight of the composition.

The surface-reacted calcium carbonate can consist of only one type of surface-reacted calcium carbonate or can be a mixture of two or more types of surface-reacted calcium carbonate. The oral care composition of the present invention may contain the surface-reacted calcium carbonate as the only remineralisation and/or whitening agent. Alternatively, the oral care composition of the present invention may contain the surface-reacted calcium carbonate in combination with at least one additional remineralisation and/or whitening agent.

According to one embodiment, the oral care composition comprises at least one additional remineralisation agent. Preferably, the additional remineralisation agent selected from the group consisting of hydroxylapatite, e.g. nano-hydroxylapatite, calcium carbonate, e.g. amorphous calcium carbonate, and combinations thereof with casein phospholipids, and mixtures thereof. Amorphous calcium carbonate is an amorphous and least stable polymorph of calcium carbonate and aside from several specialized organisms it is not found naturally.

According to another embodiment, the oral care composition comprises at least one additional whitening agent. The additional whitening agent can be a bleaching agent, an abrasive, or a remineralisation agent, and is preferably selected from the group consisting of hydrogen peroxide, carbamide peroxide, hydroxylapatite, calcium carbonate, fluoride compounds, and mixtures thereof.

According to one embodiment of the present invention, the at least one additional remineralisation and/or whitening agent is selected from the group consisting of hydroxylapatite, e.g. nano-hydroxylapatite, calcium carbonate, e.g. amorphous calcium carbonate, and combinations thereof with casein phospholipids, hydrogen peroxide, carbamide peroxide, fluoride compounds, and mixtures thereof.

According to one embodiment, the additional remineralisation and/or whitening agent has a weight median particle size *d*₅₀ from 10 nm to 100 µm, preferably from 0.1 to 50 µm, more preferably from 1 to 20 µm, and most preferably from 2 to 10 µm.

The at least one additional remineralisation and/or whitening agent can be present in the oral care composition in an amount from 1 to 20 wt.-%, preferably from 1.5 to 15 wt.-%, more preferably from 2 to 10 wt.-%, based on the total weight of the composition.

According to one embodiment, the oral care composition of the present invention comprises from 1 to 40 wt.-% of the surface-reacted calcium carbonate and from 1 to 20 wt.-% of an additional remineralisation and/or whitening agent, based on the total weight of the composition.

The oral care composition of the present invention can be, for example, a toothpaste, a toothpowder, a varnish, an adhesive gel, a cement, a resin, a spray, a foam, a balm, a composition carried out on a mouthstrip or a buccal adhesive patch, a chewable tablet, a chewable pastille, a chewable gum, a lozenge, a beverage, or a mouthwash.

According to one embodiment of the present invention, the oral care composition is a toothpaste, a toothpowder, or a mouthwash, and preferably a toothpaste.

According to a preferred embodiment, the oral care composition is a toothpaste, a toothpowder, or a mouthwash and the surface-reacted calcium carbonate is a reaction product of natural or synthetic calcium carbonate with carbon dioxide and phosphoric acid. According to another preferred embodiment, the oral care composition is a toothpaste, a toothpowder, or a mouthwash and the surface-reacted calcium carbonate is a reaction product of natural or synthetic calcium carbonate with carbon dioxide and phosphoric acid, wherein the surface-reacted calcium carbonate is in form of particles having a volume median grain diameter (*d*₅₀) of equal to or less than 15 µm, preferably from 1 to 10 µm, more preferably from 2 to 8 µm, and most preferably from 3 to 7 µm, and/or a volume determined top cut particle size (*d*₉₈) of equal to or less than 25 µm, preferably from 7 to 22 µm, more preferably from 10 to 20, and most preferably from 15 to 18 µm.

According to one embodiment of the present invention, the oral care composition has a pH between 7.5 and 10, preferably between 8 and 9.

The surface-reacted calcium carbonate can be used in combination with a fluoride compound. The inventors surprisingly found that a combination of surface-reacted calcium carbonate and a fluoride compound leads to improved remineralisation and/or whitening of teeth.

According to a preferred embodiment, the oral composition further comprises a fluoride compound. The fluoride compound can be selected from the group consisting of sodium fluoride, stannous fluoride, sodium monofluorophosphate, potassium fluoride, potassium stannous fluoride, sodium fluorostannate, stannous chlorofluoride, amine fluoride, and mixtures thereof. Preferably, the fluoride compound is sodium monofluorophosphate and/or sodium fluoride. Good results can be achieved by employing an amount of fluoride compound to provide available fluoride ion in the range of 300 to 2 000 ppm in the oral care composition, preferably about 1 450 ppm.

According to one embodiment, an oral care composition, preferably a toothpaste, a toothpowder, or a mouthwash, for use in remineralisation and/or whitening of teeth is provided, comprising a surface-reacted calcium carbonate, wherein the surface-reacted calcium carbonate is a reaction product of natural or synthetic calcium carbonate with carbon dioxide and at least one acid, preferably phosphoric acid, and wherein the oral composition further comprises a fluoride compound, preferably selected from the group consisting of sodium fluoride, stannous fluoride, sodium monofluorophosphate, potassium fluoride, potassium stannous fluoride, sodium fluorostannate, stannous chlorofluoride, amine fluoride, and mixtures thereof, and more preferably selected from sodium monofluorophosphate and/or sodium fluoride.

In addition to the surface-reacted calcium carbonate, the optional additional remineralisation and/or whitening agent, and the optional fluoride compound, the oral care composition may further comprise bioadhesive polymers, surfactants, binders, humectants, desensitising agents, flavouring agents, sweetening agents and/or water.

According to one embodiment of the present invention, the oral care composition comprises a bioadhesive polymer. The bioadhesive polymer may include any polymer that promotes adhesion of the surface-reacted calcium carbonate to teeth or tooth surface and remains on the teeth or tooth surface for an extended period of time, for example, 1 hour, 3 hours, 5 hours, 10 hours, 24 hours. In certain embodiments, the bioadhesive polymer may become more adhesive when the oral care composition is moistened with, for example, water or saliva. In other embodiments, the bioadhesive polymer is a material or combination of materials that enhance the retention of the active ingredient on the teeth or a tooth surface onto which the composition is applied. Such bioadhesive polymers include, for example, hydrophilic organic polymers, hydrophobic organic polymers, silicone gums, silicas, and combinations thereof. According to one embodiment, the bioadhesive polymer is selected from the group consisting of hydroxyethyl methacrylate, PEG/PPG copolymers, polyvinylmethylether/maleic anhydride copolymers, polyvinylpyrrolidone (PVP), cross-linked PVP, shellac, polyethylene oxide, methacrylates, acrylates copolymers, methacrylic copolymers, vinylpyrrolidone/vinyl acetate copolymers, polyvinyl caprolactum, polylactides, silicone resins, silicone adhesives, chitosan, milk proteins (casein), amelogenin, ester gum, and combinations thereof.

Suitable surfactants are generally anionic organic synthetic surfactants throughout a wide pH range. Representative of such surfactants used in the range of about 0.5 to 5 wt.-%, based on the total weight of the oral care composition, are water-soluble salts of C₁₀-C₁₈ alkyl sulphates, such as sodium lauryl sulphate, of sulphonated monoglycerides of fatty acids, such as sodium monoglyceride sulphonates, of fatty acid amides of taurine, such as sodium N-methyl-N-palmitoyltauride, and of fatty acid esters of isethionic acid, and aliphatic acylamides, such as sodium N-lauroyl sarcosinate. However, surfactants obtained from natural sources such as cocamidopropyl betaine may also be used.

Suitable binders or thickening agents to provide the desired consistency are, for example, hydroxyethylcellulose, sodium carboxymethylcellulose, natural gums, such as gum karaya, gum arabic, gum tragacanth, xanthan gum or cellulose gum, colloidal silicates, or finely divided silica. Generally, from 0.5 to 5 wt.-%, based on the total weight of the oral care composition, can be used.

Desensitising agents can be selected from the group consisting of potassium nitrate, gluteraldehyde, silver nitrate, zinc chloride, strontium chloride hexahydrate, sodium fluoride, stannous fluoride, strontium chloride, strontium acetate, arginine, hydroxylapatite, calcium sodium phosphosilicate, potassium oxalate, calcium phosphate, calcium carbonate, bioactive glasses, and mixtures thereof.

Various humectants known to the skilled person can be used, such as glycerine, sorbitol and other polyhydric alcohols, for example, in an amount from 20 to 40 wt.-%, based on the total weight of the oral care composition. Examples of suitable flavouring agents include oil of wintergreen, oil of spearmint, oil of peppermint, oil of clove, oil of sassafras and the like. Saccharin, aspartame, dextrose, or levulose can be used as sweetening agents, for example, in an amount from 0.01 to 1 wt.-%, based on the total weight of the oral care composition. Preservatives such as sodium benzoate may be present in an amount from 0.01 to 1 wt.-%, based on the total weight of the oral care composition. Colorants such as titanium dioxide may also be added to the oral care composition, for example, in an amount from 0.01 to 1.5 wt.-%, based on the total weight of the oral care composition.

The oral care composition of the present invention may also contain a material selected from the group consisting of silica, precipitated silica, alumina, aluminosilicate, metaphosphate, tricalcium phosphate, calcium pyrophosphate, ground calcium carbonate, precipitated calcium carbonate, sodium bicarbonate, bentonite, kaolin, aluminium hydroxide, calcium hydrogen phosphate, hydroxylapatite, and mixtures thereof. Said material may be present in an amount from 1 to 40 wt.-%, based on the total weight of the oral care composition. According to one embodiment, the oral care composition contains a material being selected from ground calcium carbonate and/or precipitated silica. According to another embodiment, the oral care composition contains a material being selected from the group consisting of ground calcium carbonate, precipitated calcium carbonate, aluminium hydroxide, calcium hydrogen phosphate, silica, hydroxylapatite, and mixtures thereof. According to a preferred embodiment of the present invention, the oral care composition comprises surface-reacted calcium carbonate, wherein the surface-reacted calcium carbonate is a reaction product of natural or synthetic calcium carbonate with carbon dioxide and at least one acid, and calcium carbonate, preferably ground calcium carbonate and/or precipitated calcium carbonate.

According to one embodiment of the present invention, the oral care composition is a tooth paste. The toothpaste may be produced by a method comprising the following steps:
I) providing a mixture of water and a humectants, and optionally at least one of a thickener, a preservative, a fluoride, and a sweetener,
II) adding a surface-reacted calcium carbonate, and optionally a colorant, to the mixture of step I), wherein the surface-reacted calcium carbonate is a reaction product of natural or synthetic calcium carbonate with carbon dioxide and at least one acid,
III) adding a surfactant to the mixture of step II), and
IV) optionally, adding a flavouring agent to the mixture of step III). However, a toothpaste of the present invention may also be produced by any other method known to the skilled person.

### Therapeutic use

It was found that surface-reacted calcium carbonate can be used in remineralisation and/or whitening of teeth. According to one embodiment of the present invention, a surface-reacted calcium carbonate for use in remineralisation of teeth is provided, wherein the surface-reacted calcium carbonate is a reaction product of natural or synthetic calcium carbonate with carbon dioxide and at least one acid. According to another embodiment of the present invention, a surface-reacted calcium carbonate for use in whitening of teeth enamel is provided, wherein the surface-reacted calcium carbonate is a reaction product of natural or synthetic calcium carbonate with carbon dioxide and at least one acid. According to still another embodiment of the present invention, a surface-reacted calcium carbonate for use in remineralisation and whitening of teeth is provided, wherein the surface-reacted calcium carbonate is a reaction product of natural or synthetic calcium carbonate with carbon dioxide and at least one acid.

According to a further aspect of the present invention, an oral care composition for use in remineralisation and/or whitening of teeth is provided, comprising a surface-reacted calcium carbonate, wherein the surface-reacted calcium carbonate is a reaction product of natural or synthetic calcium carbonate with carbon dioxide and at least one acid. According to one embodiment, an oral care composition for use in remineralisation of teeth is provided, comprising a surface-reacted calcium carbonate, wherein the surface-reacted calcium carbonate is a reaction product of natural or synthetic calcium carbonate with carbon dioxide and at least one acid. According to another embodiment, an oral care composition for use in whitening of teeth enamel is provided, comprising a surface-reacted calcium carbonate, wherein the surface-reacted calcium carbonate is a reaction product of natural or synthetic calcium carbonate with carbon dioxide and at least one acid. According to still another embodiment, an oral care composition for use in remineralisation and whitening of teeth is provided, comprising a surface-reacted calcium carbonate, wherein the surface-reacted calcium carbonate is a reaction product of natural or synthetic calcium carbonate with carbon dioxide and at least one acid.

The inventors of the present invention surprisingly found that surface-reacted calcium carbonate is useful in remineralisation and/or whitening of teeth. Surface-reacted calcium carbonate differs from conventional calcium carbonate in several aspects. For example, unlike conventional calcium carbonate, surface-reacted calcium carbonate comprises a porous, platy or lamellar surface structure. Without being bound to any theory, it is believed that during application of the surface-reacted calcium carbonate, for example, on the tooth of a patient, the surface-reacted calcium carbonate breaks into pieces, whereby the porous platy or lamellar surface structure elements are cleaved from the surface of the surface-reacted calcium carbonate. Said cleaved porous platy or lamellar surface structure elements may provide an improved adherence to the teeth enamel.

Furthermore, the surface treatment renders the surface-reacted calcium carbonate more resistant against acids. Therefore, the surface-reacted calcium carbonate may be more stable under acidic conditions, for example, during consumption of acidic beverages such as soft drinks or acidic dishes such as salads with vinegar-based dressings. Another advantage of the surface-reacted calcium carbonate of the present invention is that it can be used in a micrometer particle size range, and thus, the use of nanosized particles can be avoided.

The surface-reacted calcium carbonate of the present invention and/or oral compositions comprising the same may be used in professional, in-office treatment or in at home treatment.

According to one embodiment, the surface-reacted calcium carbonate for use in remineralisation and/whitening of teeth is used in a method comprising the step of administering to at least one tooth of a patient a therapeutically effective amount of the surface-reacted calcium carbonate at least once a day, preferably twice a day and more preferably three-times a day. A "therapeutically effective" amount of the surface-reacted calcium carbonate is an amount that is sufficient to have the desired therapeutic or prophylactic effect in the human subject to whom the active agent is administered, without undue adverse side effects (such as toxicity, irritation, or allergic response), commensurate with a reasonable benefit/risk ratio when used in the manner of this invention. The specific effective amount will vary with such factors as the particular condition being treated, the physical condition of the subject, the nature of concurrent therapy (if any), the specific dosage form, the oral care composition employed, and the desired dosage regimen.

According to one embodiment, the oral composition for use in remineralisation and/or whitening of teeth is used in a method comprising the step of applying the composition to at least one tooth of a patient for an effective amount of time, preferably the composition remains on the at least one tooth for at least 1 min, at least 15 min, at least 30 min, at least 1 hour, at least 2 hours, at least 12 hours or at least 24 hours.

The surface-reacted calcium carbonate of the present invention or the oral composition comprising the surface-reacted calcium carbonate of the present invention may be effective for whitening of teeth even in the absence of any oxidative whitening compound. According to a preferred embodiment of the present invention, the oral care composition does not contain an oxidative whitening compound.

According to one embodiment, the surface-reacted calcium carbonate of the present invention or the oral composition comprising the surface-reacted calcium carbonate of the present invention is used in a cosmetic method for whitening teeth, comprising the step of applying the composition to at least one tooth of an individual for an effective amount of time, preferably the composition remains on the at least one tooth for at least 1 min, at least 15 min, at least 30 min, at least 1 hour, at least 2 hours, at least 12 hours or at least 24 hours.

The scope and interest of the present invention will be better understood based on the following figures and examples which are intended to illustrate certain embodiments of the present invention and are non-limitative.

### Description of the figure:

Fig. 1 shows a plot of the results of the surface micro hardness (SMH) measurements for the prepared toothpaste samples of Example 1.
Fig. 2 shows a graph of the values of the CIELAB L* coordinates determined for the prepared toothpaste samples of Example 1.
Fig. 3 shows a graph of the values of the CIELAB b* coordinates determined for the prepared toothpaste samples of Example 1.
Fig. 4 shows a scanning electron microscope (SEM) micrograph of a demineralised bovine enamel sample.
Fig. 5 shows a scanning electron microscope (SEM) micrograph of a demineralised bovine enamel sample.
Fig. 6 shows a scanning electron microscope (SEM) micrograph of a remineralised bovine enamel sample, which was treated with inventive toothpaste sample 1 of Example 1.
Fig. 7 shows a scanning electron microscope (SEM) micrograph of a remineralised bovine enamel sample, which was treated with inventive toothpaste sample 1 of Example 1.

### Examples

### 1. Measurement methods

In the following, measurement methods implemented in the examples are described.

### Particle size distribution

The particle size distribution of non surface-reacted calcium carbonate particles, e.g., ground calcium carbonate, was measured using a Sedigraph 5100 from the company Micromeritics, USA. The method and the instrument are known to the skilled person and are commonly used to determine grain size of fillers and pigments. The measurement was carried out in an aqueous solution comprising 0.1 wt.-% Na₄P₂O₇. The samples were dispersed using a high speed stirrer and supersonics. For the measurement of dispersed samples, no further dispersing agents were added.

The volume median grain diameter (*d*₅₀) of surface-reacted calcium carbonate was determined using a Malvern Mastersizer 2000 Laser Diffraction System (Malvern Instruments Plc., Great Britain).

### Specific surface area (SSA)

The specific surface area is measured via the BET method according to ISO 9277 using nitrogen, following conditioning of the sample by heating at 250°C for a period of 30 minutes. Prior to such measurements, the sample is filtered within a Büchner funnel, rinsed with deionised water and dried overnight at 90 to 100°C in an oven. Subsequently the dry cake is ground thoroughly in a mortar and the resulting powder placed in a moisture balance at 130°C until a constant weight is reached.

### Scanning electron microscope (SEM) micrographs

The prepared bovine enamel samples were examined by a Sigma VP field emission scanning electron microscope (Carl Zeiss AG, Germany) and a variable pressure secondary electron detector (VPSE) with a chamber pressure of about 50 Pa.

### 2. Materials

MCC: surface-reacted calcium carbonate based on ground calcium carbonate and phosphoric acid (*d*₅₀ = 6.54 µm, *d*₉₈ = 16.8 µm, SSA = 25.1 m²/g). The ground calcium carbonate was obtained from Orgon, France, (*d*₅₀ = 3 µm, *d*₉₈ = 12 µm) and is commercially available from Omya AG, Switzerland.

GCC: natural ground calcium carbonate obtained from Avenza-Carrara, Italy (*d*₅₀ = 5 µm, *d*₉₈ = 30 µm), commercially available from Omya AG, Switzerland.

### 3. Examples

### Example 1 - Toothpaste compositions

Toothpaste samples 1 to 4 were produced according to the following procedure using the ingredients and amounts compiled in Table 1 below.

Step A: Water and sorbitol were mixed in a beaker. Xanthan gum, sodium benzoate, sodium monofluorophosphate (phoskadent Na 211, BK Giulini, Germany) and sodium saccharine were mixed and the obtained mixture was added to the beaker.

Step B: MCC or GCC, respectively, and titanium dioxide were wetted with water, and subsequently added to the mixture of step A. The mixture was homogenized until a smooth mixture was obtained.

Step C: The silica Sorbosil TC 15 (PQ Corporation, USA) was added to the mixture of step B under homogenizing conditions, whereby the mixture was heating up strongly. The mixture was stirred until it was cooled down to room temperature.

Step D: The surfactant sodium lauryl sulphate was added in form of a 25% solution to the mixture of step C under slow agitation.

Step E: 0.8 wt.-% (2.4 g) spearmint flavour was added to the mixture of step D.

**Table 1: Ingredients and amounts of toothpaste samples 1 to 4. The percentages refer to weight percentages based on the total weight of the composition.**

| Ingredients | Sample 1 | Sample 2 | Sample 3 (comparative) | Sample 4 (comparative) |
|---|---|---|---|---|
| Sorbitol 70% | 31.0% (93.0 g) | 32.0 % (96.0 g) | 31.0 % (93.0 g) | 32.0 % (96.0 g) |
| Water | 30.0 % (90.0 g) | 30.1 % (90.3 g) | 30.5 % (91.5 g) | 30.6 % (82.2 g) |
| Phoskadent Na 211 | 1.1 % (3.3 g) | -- | 1.1 % (3.3 g) | -- |
| Xanthan gum | 0.8 % (2.4 g) | 0.8 % (2.4 g) | 0.8 % (2.4 g) | 0.8 % (2.4 g) |
| Sodium saccharin | 0.1 % (0.3 g) | 0.1 % (0.3 g) | 0.1 % (0.3 g) | 0.1 % (0.3 g) |
| Sodium benzoate | -- | -- | -- | -- |
| MCC | 30.0 % (90.0 g) | 30.0 % (90.0 g) | -- | -- |
| GCC | -- | -- | 30.0 % (90.0 g) | 30.0 % (90.0 g) |
| Titanium dioxide | 2.0 % (6.0 g) | 2.0 % (6.0 g) | 0.5 % (1.5 g) | 0.5 % (1.5 g) |
| Sorbosil TC 15 | 3.0 % (9.0 g) | 3.0 % (9.0 g) | 4.0 % (12.0 g) | 3.0 % (9.0 g) |
| Sodium lauryl sulphate (25% solution) | 2.0 % (6.0 g) | 2.0 % (6.0 g) | 2.0 % (6.0 g) | 2.0 % (6.0 g) |

### Example 2 - Remineralisation study

Bovine enamel samples were prepared as follows:
Bovine enamel blocks (4 mm x 4 mm) were cut, lapped plano-parallel, and hand-polished. The mean baseline surface micro hardness (SMH) value was determined using a MicroMet 5103 Hardness testing machine with a Knoop indenter and the MicroMet MHT Software (Buehler Ltd., USA) with a 50 g load, 10 s indent time and 5 indents per block.

The bovine enamel samples were demineralised for 14 days in a 1:1 8% methyl cellulose/lactic acid gel system, at 37°C and pH 4.6. After demineralisation, the SMH of each sample was measured. The samples were stratified into cells of 10 blocks, wherein for each of the four toothpaste samples of Example 1 one cell was provided. In addition, a commercially available non-fluoride silica toothpaste sample (Boots Smile non fluoride freshmint toothpaste, commercially available from Boots UK Ltd., Great Britain) was tested as sample 5 on a further cell and three more cells were used in order to cover attrition.

The samples were subjected to a 5 min treatment with a 3:1 water:toothpaste slurry containing 5 UI/ml phosphatase, 30 min with an acidic buffer (50 mM acetic acid, 1.50 mM calcium chloride dihydrate, 0.90 mM potassium dihydrogen orthophosphate, 130 mM potassium chloride, pH 5.0) and 10 min with a neutral buffer (20 mM HEPES, 1.50 mM calcium chloride dihydrate, 0.90 mM potassium dihydrogen orthophosphate, 130 mM potassium chloride, pH 7.0). This cycle was repeated 6 times daily for 8 days.

After that cycle was finished, the samples were left in the neutral buffer overnight. Efficacy was assessed by SMH analysis of the samples, before and after pH-cycling. Ten readings were taken per sample and remineralisation was expressed as a change in SMH. The CIELAB coordinates L*a*b* of all blocks at the SMH baseline measurement, after demineralisation, and after remineralisation were recorded using a CR321 Konika Minolta chromameter (Konica Minolta, Inc., Japan). In addition, the surface structure of the samples was examined by a Sigma VP field emission scanning electron microscope (Carl Zeiss AG, Germany) and a variable pressure secondary electron detector (VPSE) with a chamber pressure of about 50 Pa.

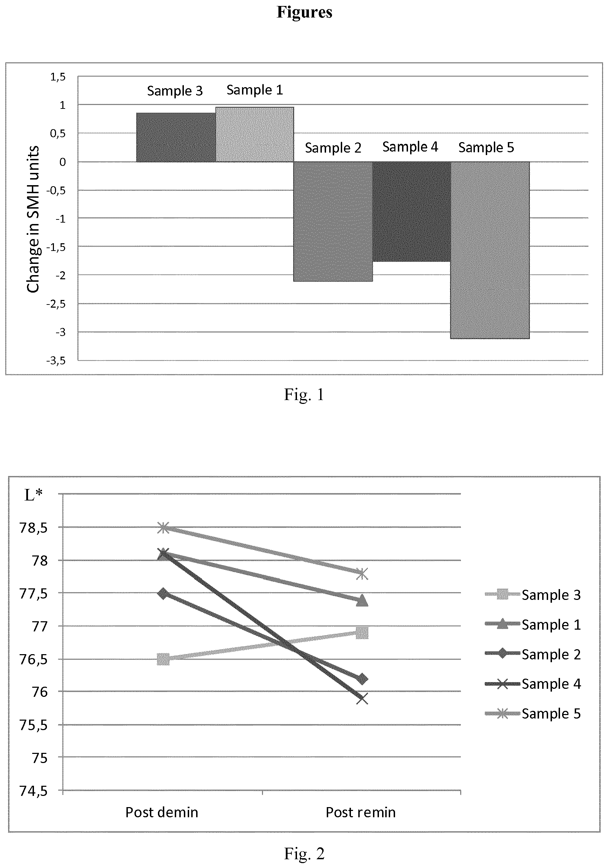

The results of the remineralisation study are shown in Figs. 1 to 7.

It can be gathered from the results of the surface micro hardness (SMH) measurements shown in Fig. 1 that both of the fluorinated toothpaste samples (sample 1 and 3) promoted remineralisation to a significantly greater extent than the non-fluoride control formulations.

The measurement of the CIELAB coordinates revealed that after demineralisation, the L* value of all tested samples was increased (post demin: after demineralisation; post remin: after remineralisation). This is caused by the decalcification of the enamel, wherein so-called white spot lesions are developed. With the exception of toothpaste sample 3, the L* value (cube of the luminance) of all tested samples decreased after remineralisation (see Fig. 2), which is an evidence for remineralisation.

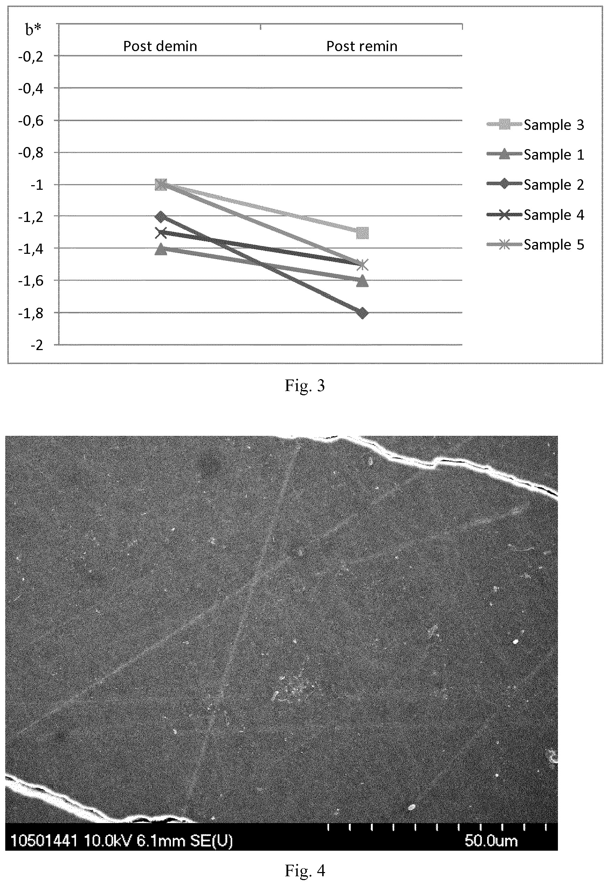

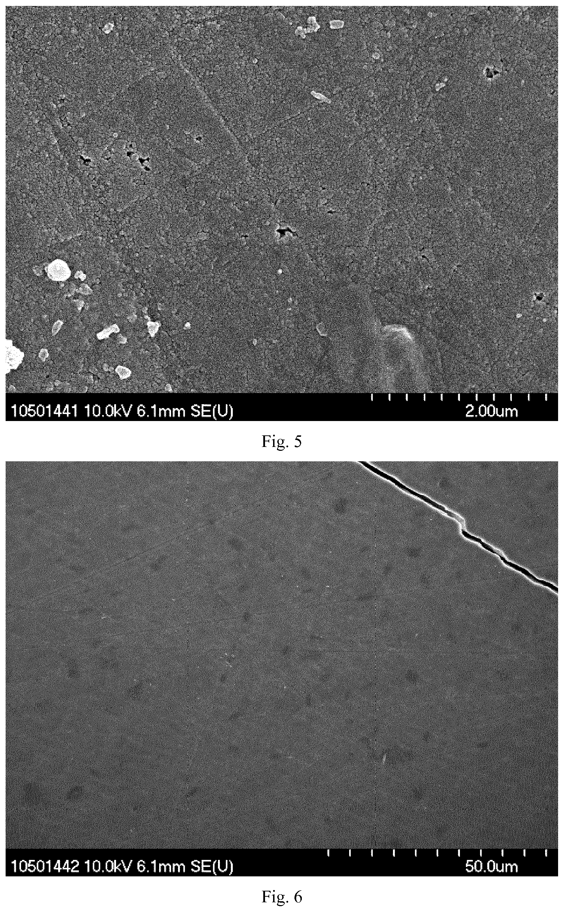

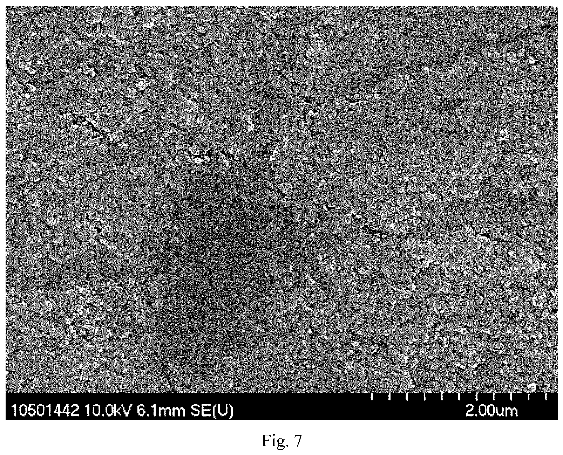

The measurement of the b* coordinate shown in Fig. 3, revealed that all enamel samples had a negative b* value (post demin: after demineralisation; post remin: after remineralisation). A negative b* value means that the recorded picture has a blue cast, which means that all enamel samples exhibited a bluish white, which is usually perceived as a very bright white. The enamel sample that was remineralised with a toothpaste containing surface-reacted calcium carbonate and no fluoride (sample 2) showed the most negative b* value, and thus, the best whitening effect. Figs. 4 and 5 show scanning electron microscope (SEM) micrographs of an enamel sample after demineralisation, and Figs. 6 and 7 shows SEM micrographs of enamel samples, which were treated with toothpaste sample 1. While cracks and an unevenness of the enamel surface is clearly visible in Figs. 4 and 5, Figs. 6 and 7 evidence that the enamel surface looks smoother and more even after the remineralisation with the inventive toothpaste.

## Claims

1. A surface-reacted calcium carbonate for use in remineralisation and/or whitening of teeth,
wherein the surface-reacted calcium carbonate is a reaction product of natural or synthetic calcium carbonate with carbon dioxide and at least one acid.

2. The surface-reacted calcium carbonate for the use according to claim 1 or 2, wherein the at least one acid is selected from the group consisting of hydrochloric acid, sulphuric acid, sulphurous acid, phosphoric acid, citric acid, oxalic acid, acetic acid, formic acid, and mixtures thereof, preferably the at least one acid is selected from the group consisting of hydrochloric acid, sulphuric acid, sulphurous acid, phosphoric acid, oxalic acid, and mixtures thereof, and more preferably the at least one acid is phosphoric acid.

3. The surface-reacted calcium carbonate for the use according to any one of the preceding claims, wherein the surface-reacted calcium carbonate is in form of particles having a volume median grain diameter (*d*₅₀) of equal to or less than 15 µm, preferably from 1 to 10 µm, more preferably from 2 to 8 µm, and most preferably from 3 to 7 µm, and/or a volume determined top cut particle size (*d*₉₈) of equal to or less than 25 µm, preferably from 7 to 22 µm, more preferably from 10 to 20, and most preferably from 15 to 18 µm.

4. The surface-reacted calcium carbonate for the use according to any one of the preceding claims, wherein the surface-reacted calcium carbonate is in form of particles having a specific surface area of from 5 m²/g to 200 m²/g, more preferably 20 m²/g to 80 m²/g, and even more preferably 30 m²/g to 60 m²/g, measured using nitrogen and the BET method according to ISO 9277.

5. The surface-reacted calcium carbonate for the use according to any one of the preceding claims, wherein the surface-reacted calcium carbonate is used in combination with a fluoride compound, preferably selected from the group consisting of sodium fluoride, stannous fluoride, sodium monofluorophosphate, potassium fluoride, potassium stannous fluoride, sodium fluorostannate, stannous chlorofluoride, amine fluoride, and mixtures thereof, and more preferably the fluoride compound is sodium monofluorophosphate and/or sodium fluoride.

6. The surface-reacted calcium carbonate for the use according to any one of the preceding claims, wherein at least one active agent is associated with the surface-reacted calcium carbonate, preferably the active agent is at least one additional desensitizing agent, and more preferably the at least one additional desensitizing agent is selected from the group consisting of potassium nitrate, gluteraldehyde, silver nitrate, zinc chloride, strontium chloride hexahydrate, sodium fluoride, stannous fluoride, strontium chloride, strontium acetate, arginine, hydroxylapatite, calcium sodium phosphosilicate, potassium oxalate, calcium phosphate, calcium carbonate, bioactive glasses, and mixtures thereof.

7. The surface-reacted calcium carbonate for the use according to any one of the preceding claims, wherein the surface-reacted calcium carbonate is obtained by a process comprising the steps of:
a) providing a suspension of natural or synthetic calcium carbonate,
b) adding at least one acid having a pKₐ value of 0 or less at 20°C or having a pKₐ value from 0 to 2.5 at 20°C to the suspension of step a), and
c) treating the suspension of step a) with carbon dioxide before, during or after step b).

8. The surface-reacted calcium carbonate for the use according to any one of the preceding claims, wherein the surface-reacted calcium carbonate is obtained by a process comprising the steps of:
A) providing a natural or synthetic calcium carbonate,
B) providing at least one water-soluble acid,
C) providing gaseous CO₂,
D) contacting said natural or synthetic calcium carbonate of step A) with the at least one acid of step B) and with the CO₂ of step C),
**characterised in that**:
i) the at least one acid of step B) has a pKₐ of greater than 2.5 and less than or equal to 7 at 20°C, associated with the ionisation of its first available hydrogen, and a corresponding anion is formed on loss of this first available hydrogen capable of forming a water-soluble calcium salt, and
ii) following contacting the at least one acid with natural or synthetic calcium carbonate, at least one water-soluble salt, which in the case of a hydrogen-containing salt has a pKₐ of greater than 7 at 20°C, associated with the ionisation of the first available hydrogen, and the salt anion of which is capable of forming water-insoluble calcium salts, is additionally provided.

9. An oral care composition for use in remineralisation and/or whitening of teeth,
comprising a surface-reacted calcium carbonate, wherein the surface-reacted calcium carbonate is a reaction product of natural or synthetic calcium carbonate with carbon dioxide and at least one acid.

10. The oral care composition for the use according to claim 9, wherein the oral care composition comprises from 1 to 40 wt.-%, preferably from 1.5 to 35 wt.-%, more preferably from 2 to 30 wt.-% of the surface-reacted calcium carbonate, based on the total weight of the composition.

11. The oral care composition for the use according to claim 9 or 10, wherein the oral care composition is a toothpaste, a toothpowder, or a mouthwash, and wherein preferably the surface-reacted calcium carbonate is a reaction product of natural or synthetic calcium carbonate with carbon dioxide and phosphoric acid.

12. The oral care composition for the use according to any one of claims 9 to 11, wherein the oral composition further comprises a fluoride compound, preferably the fluoride compound is selected from the group consisting of sodium fluoride, stannous fluoride, sodium monofluorophosphate, potassium fluoride, potassium stannous fluoride, sodium fluorostannate, stannous chlorofluoride, amine fluoride, and mixtures thereof, and more preferably the fluoride compound is sodium monofluorophosphate and/or sodium fluoride.

13. The oral care composition for the use according to any one of claims 9 to 12, wherein the oral care composition further comprises an additional remineralisation and/or whitening agent, preferably selected from the group consisting of hydroxylapatite, e.g. nano-hydroxylapatite, calcium carbonate, e.g. amorphous calcium carbonate, and combinations thereof with casein phospholipids, hydrogen peroxide, carbamide peroxide, fluoride compounds, and mixtures thereof.

14. The oral care composition for the use according to any one of claims 9 to 13, wherein the oral care composition has a pH between 7.5 and 10, preferably between 8 and 9.

15. A surface-reacted calcium carbonate for use in remineralisation of teeth,
wherein the surface-reacted calcium carbonate is a reaction product of natural or synthetic calcium carbonate with carbon dioxide and at least one acid.

16. A surface-reacted calcium carbonate for use in whitening of teeth enamel,
wherein the surface-reacted calcium carbonate is a reaction product of natural or synthetic calcium carbonate with carbon dioxide and at least one acid.

## Patentansprüche

1. Calciumcarbonat, das an der Oberfläche eine Reaktion eingegangen ist, für die Verwendung bei der Remineralisierung oder beim Bleichen von Zähnen, wobei das Calciumcarbonat, das an der Oberfläche eine Reaktion eingegangen ist, ein Reaktionsprodukt von natürlichem oder synthetischem Calciumcarbonat mit Kohlenstoffdioxid und wenigstens einer Säure ist.

2. Calciumcarbonat, das an der Oberfläche eine Reaktion eingegangen ist, für die Verwendung nach Anspruch 1 oder 2, wobei die wenigstens eine Säure ausgewählt ist aus der Gruppe bestehend aus Salzsäure, Schwefelsäure, schwefliger Säure, Phosphorsäure, Zitronensäure, Oxalsäure, Essigsäure, Ameisensäure, sowie Gemischen davon, bevorzugt ist die wenigstens eine Säure ausgewählt aus der Gruppe bestehend aus Salzsäure, Schwefelsäure, schwefliger Säure, Phosphorsäure, Oxalsäure, sowie Gemischen davon, und bevorzugter ist die wenigstens eine Säure Phosphorsäure.

3. Calciumcarbonat, das an der Oberfläche eine Reaktion eingegangen ist, für die Verwendung nach einem der vorhergehenden Ansprüche, wobei das Calciumcarbonat, das an der Oberfläche eine Reaktion eingegangen ist, in der Form von Partikeln mit einem volumengemittelten Korndurchmesser (*d*₅₀) von gleich oder kleiner 15 µm ist, bevorzugt von 1 bis 10 µm, bevorzugter von 2 bis 8 µm, und am meisten bevorzugt von 3 bis 7 µm, und/oder einer volumenbestimmten Topcut-Partikelgrösse (*d*₉₈) von gleich oder kleiner 25 µm, bevorzugt von 7 bis 22 µm, bevorzugter von 10 bis 20 µm, und am meisten bevorzugt von 15 bis 18 µm.

4. Calciumcarbonat, das an der Oberfläche eine Reaktion eingegangen ist, für die Verwendung nach einem der vorhergehenden Ansprüche, wobei das Calciumcarbonat, das an der Oberfläche eine Reaktion eingegangen ist, in der Form von Partikeln mit einer spezifischen Oberfläche von 5 m²/g bis 200 m²/g ist, bevorzugter 20 m²/g bis 80 m²/g, und noch bevorzugter 30 m²/g bis 60 m²/g, gemessen durch Verwenden von Stickstoff und dem BET-Verfahren gemäss ISO 9277.

5. Calciumcarbonat, das an der Oberfläche eine Reaktion eingegangen ist, für die Verwendung nach einem der vorhergehenden Ansprüche, wobei das Calciumcarbonat, das an der Oberfläche eine Reaktion eingegangen ist, in Kombination mit einer Fluoridverbindung verwendet wird, bevorzugt ausgewählt aus der Gruppe bestehend aus Natriumfluorid, Zinnfluorid, Natriummonofluorphosphat, Kaliumfluorid, Kaliumzinnfluorid, Natriumfluorstannat, Zinnchlorfluorid, Aminfluorid, sowie Gemischen davon, und bevorzugter ist die Fluoridverbindung Natriummonofluorphosphat und/oder Natriumfluorid.

6. Calciumcarbonat, das an der Oberfläche eine Reaktion eingegangen ist, für die Verwendung nach einem der vorhergehenden Ansprüche, wobei wenigstens ein Wirkstoff mit dem Calciumcarbonat, das an der Oberfläche eine Reaktion eingegangen ist, assoziiert ist, bevorzugt ist der Wirkstoff wenigstens ein zusätzliches Desensibilisierungsmittel, und bevorzugter ist das wenigstens eine zusätzliche Desensibilisierungsmittel ausgewählt aus der Gruppe bestehend aus Kaliumnitrat, Glutaraldehyd, Silbernitrat, Zinkchlorid, Strontiumchloridhexahydrat, Natriumfluorid, Zinnfluorid, Strontiumchlorid, Strontiumacetat, Arginin, Hydroxyapatit, Calciumnatriumphosphosilikat, Kaliumoxalat, Calciumphosphat, Calciumcarbonat, bioaktiven Gläsern, sowie Gemischen davon.

7. Calciumcarbonat, das an der Oberfläche eine Reaktion eingegangen ist, für die Verwendung nach einem der vorhergehenden Ansprüche, wobei das Calciumcarbonat, das an der Oberfläche eine Reaktion eingegangen ist, erhalten wurde durch ein Verfahren umfassend die Schritte:
a) Bereitstellen von einer Suspension von natürlichem oder synthetischem Calciumcarbonat,
b) Zugeben von wenigstens einer Säure mit einem pKₐ-Wert von 0 oder kleiner bei 20°C, oder mit einem pKₐ-Wert von 0 bis 2,5 bei 20°C, zu der Suspension von Schritt a), und
c) Behandeln der Suspension von Schritt a) mit Kohlenstoffdioxid vor, während oder nach Schritt b).

8. Calciumcarbonat, das an der Oberfläche eine Reaktion eingegangen ist, für die Verwendung nach einem der vorhergehenden Ansprüche, wobei das Calciumcarbonat, das an der Oberfläche eine Reaktion eingegangen ist, erhalten wurde durch ein Verfahren umfassend die Schritte:
A) Bereitstellen von einem natürlichen oder synthetischen Calciumcarbonat,
B) Bereitstellen von wenigstens einer wasserlöslichen Säure,
C) Bereitstellen von gasförmigem CO₂,
D) Inkontaktbringen des natürlichen oder synthetischen Calciumcarbonats von Schritt A) mit der wenigstens einen Säure von Schritt B) und mit dem CO₂ von Schritt C),
**gekennzeichnet dadurch, dass**:
i) die wenigstens eine Säure von Schritt B) einen pKₐ von grösser 2,5 und kleiner oder gleich 7 bei 20°C aufweist, assoziiert mit der Ionisierung ihres ersten verfügbaren Wasserstoffs, und ein korrespondierendes Anion wird gebildet beim Verlust dieses ersten verfügbaren Wasserstoffs, das imstande ist wasserlösliche Calciumsalze zu bilden;
ii) im Anschluss an das Inkontaktbringen der wenigstens einen Säure mit natürlichem oder synthetischen Calciumcarbonat werden wenigstens ein wasserlösliches Salz, welches im Fall eines Wasserstoff enthaltenden Salzes einen pKₐ von grösser 7 bei 20°C aufweist, assoziiert mit der Ionisierung ihres ersten verfügbaren Wasserstoffs, und das Salzanion, das imstande ist wasserunlösliche Calciumsalze zu bilden, zusätzlich bereitgestellt.

9. Mundpflegezusammensetzung für die Verwendung bei der Remineralisierung oder beim Bleichen von Zähnen, umfassend ein Calciumcarbonat, das an der Oberfläche eine Reaktion eingegangen ist,
wobei das Calciumcarbonat, das an der Oberfläche eine Reaktion eingegangen ist, ein Reaktionsprodukt von natürlichem oder synthetischem Calciumcarbonat mit Kohlenstoffdioxid und wenigstens einer Säure ist.

10. Mundpflegezusammensetzung für die Verwendung nach Anspruch 9, wobei die Mundpflegezusammensetzung von 1 bis 40 Gew.-%, bevorzugt von 1,5 bis 35 Gew.-%, bevorzugter von 2 bis 30 Gew.-% des Calciumcarbonat, das an der Oberfläche eine Reaktion eingegangen ist, umfasst, bezogen auf das Gesamtgewicht der Zusammensetzung.

11. Mundpflegezusammensetzung für die Verwendung nach Anspruch 9 oder 10, wobei die Mundpflegezusammensetzung eine Zahnpasta, ein Zahnpulver oder ein Mundwasser ist, und wobei bevorzugt das Calciumcarbonat, das an der Oberfläche eine Reaktion eingegangen ist, ein Reaktionsprodukt eines natürlichen oder synthetischen Calciumcarbonats mit Kohlenstoffdioxid und Phosphorsäure ist.

12. Mundpflegezusammensetzung für die Verwendung nach einem der Ansprüche 9 bis 11, wobei die Mundpflegezusammensetzung ferner eine Fluoridverbindung umfasst, bevorzugt ist die Fluoridverbindung ausgewählt aus der Gruppe bestehend aus Natriumfluorid, Zinnfluorid, Natriummonofluorphosphat, Kaliumfluorid, Kaliumzinnfluorid, Natriumfluorstannat, Zinnchlorfluorid, Aminfluorid, sowie Gemischen davon, und bevorzugter ist die Fluoridverbindung Natriummonofluorphosphat und/oder Natriumfluorid.

13. Mundpflegezusammensetzung für die Verwendung nach einem der Ansprüche 9 bis 12, wobei die Mundpflegezusammensetzung ferner ein zusätzliches Remineralisierungs- und/oder Bleichmittel umfasst, bevorzugt ausgewählt aus der Gruppe bestehend aus Hydroxyapatit, z.B. Nano-Hydroxyapatit, Calciumcarbonat, z.B. amorphes Calciumcarbonat, sowie Kombinationen davon mit Casein-Phospholipiden, Wasserstoffperoxid, Carbamidperoxid, Fluoridverbindungen, sowie Gemischen davon.

14. Mundpflegezusammensetzung für die Verwendung nach einem der Ansprüche 9 bis 13, wobei die Mundpflegezusammensetzung einen pH zwischen 7,5 und 10 aufweist, bevorzugt zwischen 8 und 9.

15. Calciumcarbonat, das an der Oberfläche eine Reaktion eingegangen ist, für die Verwendung bei der Remineralisierung von Zähnen,
wobei das Calciumcarbonat, das an der Oberfläche eine Reaktion eingegangen ist, ein Reaktionsprodukt von natürlichem oder synthetischem Calciumcarbonat mit Kohlenstoffdioxid und wenigstens einer Säure ist.

16. Calciumcarbonat, das an der Oberfläche eine Reaktion eingegangen ist, für die Verwendung beim Bleichen von Zahnschmelz,
wobei das Calciumcarbonat, das an der Oberfläche eine Reaktion eingegangen ist, ein Reaktionsprodukt von natürlichem oder synthetischem Calciumcarbonat mit Kohlenstoffdioxid und wenigstens einer Säure ist.

## Revendications

1. Carbonate de calcium ayant réagi en surface pour une utilisation dans la reminéralisation et/ou le blanchissement des dents,
dans lequel le carbonate de calcium ayant réagi en surface est un produit de réaction de carbonate de calcium naturel ou synthétique avec du dioxyde de carbone et au moins un acide.

2. Carbonate de calcium ayant réagi en surface pour une utilisation selon la revendication 1 ou 2, dans lequel l'au moins un acide est choisi dans le groupe constitué par l'acide chlorhydrique, l'acide sulfurique, l'acide sulfureux, l'acide phosphorique, l'acide citrique, l'acide oxalique, l'acide acétique, l'acide formique et des mélanges de ceux-ci, de préférence l'au moins un acide est choisi dans le groupe constitué par l'acide chlorhydrique, l'acide sulfurique, l'acide sulfureux, l'acide phosphorique, l'acide oxalique et des mélanges de ceux-ci, et plus préférablement l'au moins un acide est l'acide phosphorique.

3. Carbonate de calcium ayant réagi en surface selon l'une quelconque des revendications précédentes, dans lequel le carbonate de calcium ayant réagi en surface est sous la forme de particules ayant un diamètre de grain médian en volume (*d₅₀*) inférieur ou égal à 15 µm, de préférence de 1 à 10 µm, plus préférablement de 2 à 8 µm, et de manière préférée entre toutes de 3 à 7 µm, et/ou une taille de particules de dessus déterminée en volume (*d₉₈*) inférieure ou égale à 25 µm, de préférence de 7 à 22 µm, plus préférablement de 10 à 20 µm, et de manière préférée entre toutes de 15 à 18 µm.

4. Carbonate de calcium ayant réagi en surface selon l'une quelconque des revendications précédentes, dans lequel le carbonate de calcium ayant réagi en surface est sous la forme de particules ayant une surface spécifique de 5 m²/g à 200 m²/g, plus préférablement de 20 m²/g à 80 m²/g et encore plus préférablement de 30 m²/g à 60 m²/g, mesurée en utilisant de l'azote et la méthode BET selon la norme ISO 9277.

5. Carbonate de calcium ayant réagi en surface selon l'une quelconque des revendications précédentes, dans lequel le carbonate de calcium ayant réagi en surface est utilisé en combinaison avec un composé à base de fluorure, de préférence choisi dans le groupe constitué par le fluorure de sodium, le fluorure stanneux, le monofluorophosphate de sodium, le fluorure de potassium, le fluorure stanneux de potassium, le fluorostannate de sodium, le chlorofluorure stanneux, le fluorure d'amine et des mélanges de ceux-ci, et plus préférablement le composé à base de fluorure est le monofluorophosphate de sodium et/ou le fluorure de sodium.

6. Carbonate de calcium ayant réagi en surface selon l'une quelconque des revendications précédentes, dans lequel l'au moins un agent actif est associé au carbonate de calcium ayant réagi en surface, de préférence l'agent actif est au moins un agent de désensibilisation supplémentaire et plus préférablement l'au moins un agent de désensibilisation supplémentaire est choisi dans le groupe constitué par le nitrate de potassium, le glutéraldéhyde, le nitrate d'argent, le chlorure de zinc, le chlorure de strontium hexahydraté, le fluorure de sodium, le fluorure stanneux, le chlorure de strontium, l'acétate de strontium, l'arginine, l'hydroxylapatite, le phosphosilicate de calcium sodique, l'oxalate de potassium, le phosphate de calcium, le carbonate de calcium, les verres bioactifs et des mélanges de ceux-ci.

7. Carbonate de calcium ayant réagi en surface selon l'une quelconque des revendications précédentes, dans lequel le carbonate de calcium ayant réagi en surface est obtenu par un procédé comprenant les étapes consistant à:
a) utiliser une suspension de carbonate de calcium synthétique ou naturel,
b) ajouter au moins un acide ayant une valeur de pKₐ inférieure ou égale à 0 à 20 °C ou ayant une valeur de pKₐ de 0 à 2,5 à 20 °C à la suspension de l'étape a), et
c) traiter la suspension de l'étape a) avec du dioxyde de carbone avant, pendant ou après l'étape b).

8. Carbonate de calcium ayant réagi en surface selon l'une quelconque des revendications précédentes, dans lequel le carbonate de calcium ayant réagi en surface est obtenu par un procédé comprenant les étapes consistant à:
A) utiliser un carbonate de calcium synthétique ou naturel,
B) utiliser au moins un acide soluble dans l'eau,
C) utiliser du CO₂ gazeux,
D) mettre en contact ledit carbonate de calcium synthétique ou naturel de l'étape A) avec l'au moins un acide de l'étape B) et avec le CO₂ de l'étape C),
**caractérisé en ce que**:
i) l'au moins un acide de l'étape B) a un pKₐ supérieur à 2,5 et inférieur ou égal à 7 à 20 °C, associé à l'ionisation de son premier atome d'hydrogène disponible, et un anion correspondant est formé lors de la perte de ce premier atome d'hydrogène disponible capable de former un sel de calcium soluble dans l'eau, et
ii) après la mise en contact de l'au moins un acide avec du carbonate de calcium naturel ou synthétique, au moins un sel soluble dans l'eau, qui dans le cas d'un sel contenant de l'hydrogène a un pKₐ supérieur à 7 à 20 °C, associé à l'ionisation du premier atome d'hydrogène disponible, et dont l'anion du sel est capable de former des sels de calcium insolubles dans l'eau, est en outre utilisé.

9. Composition de soin oral pour une utilisation dans la reminéralisation et/ou le blanchiment des dents,
comprenant un carbonate de calcium ayant réagi en surface, le carbonate de calcium ayant réagi en surface étant un produit de réaction de carbonate de calcium naturel ou synthétique avec du dioxyde de carbone et au moins un acide.

10. Composition de soin oral pour une utilisation selon la revendication 9, dans laquelle la composition de soin oral comprend de 1 à 40 % en poids, de préférence de 1,5 à 35 % en poids, plus préférablement de 2 à 30 % en poids du carbonate de calcium ayant réagi en surface, par rapport au poids total de la composition.

11. Composition de soin oral pour une utilisation selon la revendication 9 ou 10, dans laquelle la composition de soin oral est un dentifrice, une poudre dentifrice ou un bain de bouche, et dans laquelle de préférence le carbonate de calcium ayant réagi en surface est un produit de réaction de carbonate de calcium naturel ou synthétique avec du dioxyde de carbone et de l'acide phosphorique.

12. Composition de soin oral pour une utilisation selon l'une quelconque des revendications 9 à 11, dans laquelle la composition orale comprend en outre un composé à base de fluorure, de préférence le composé à base de fluorure est choisi dans le groupe constitué par le fluorure de sodium, le fluorure stanneux, le monofluorophosphate de sodium, le fluorure de potassium, le fluorure stanneux de potassium, le fluorostannate de sodium, le chlorofluorure stanneux, le fluorure d'amine et des mélanges de ceux-ci, et plus préférablement le composé à base de fluorure est le monofluorophosphate de sodium et/ou le fluorure de sodium.

13. Composition de soin oral pour une utilisation selon l'une quelconque des revendications 9 à 12, dans laquelle la composition de soin orale comprend en outre un agent de reminéralisation et/ou de blanchiment supplémentaire, de préférence choisi dans le groupe constitué par une hydroxylapatite, par exemple la nanohydroxylapatite, un carbonate de calcium, par exemple le carbonate de calcium amorphe, et des combinaisons de ceux-ci avec des phospholipides de caséine, du peroxyde d'hydrogène, du peroxyde de carbamide, des composés à base de fluorure, et des mélanges de ceux-ci.

14. Composition de soin oral pour une utilisation selon l'une quelconque des revendications 9 à 13, dans laquelle la composition de soin oral a un pH compris entre 7,5 et 10, de préférence entre 8 et 9.

15. Carbonate de calcium ayant réagi en surface pour une utilisation dans la reminéralisation des dents,
dans lequel le carbonate calcium ayant réagi en surface est un produit de réaction de carbonate de calcium naturel ou synthétique avec du dioxyde de carbone et au moins un acide.

16. Carbonate de calcium ayant réagi en surface pour une utilisation dans le blanchiment de l'émail dentaire,
dans lequel le carbonate calcium ayant réagi en surface est un produit de réaction de carbonate de calcium naturel ou synthétique avec du dioxyde de carbone et au moins un acide.
